# EUROPEAN PATENT SPECIFICATION

(11) **EP 4 405 323 B1**
(45) Date of publication and mention of the grant of the patent: **14.01.2026**
(21) Application number: 22822032.3
(22) Date of filing: 24.11.2022
(51) Int. Cl.: C07C 49/543, C07C 61/22, C07C 51/38, C07C 45/67

(54) **PROCESS FOR PREPARING A CYCLIC DIENE**
VERFAHREN ZUR HERSTELLUNG EINES CYCLISCHEN DIENS
PROCÉDÉ DE PRÉPARATION D'UN DIÈNE CYCLIQUE

(30) Priority: 25.11.2021 EP 21210351
(43) Date of publication of application: 31.07.2024
(73) Proprietor: Firmenich SA, 1242 Satigny (CH)
(72) Inventor: JACOBY, Denis, 1242 Satigny (CH); CHAPPUIS, Simone, 1242 Satigny (CH); KELLER, Fabrice, 1242 Satigny (CH)
(74) Representative: dsm-firmenich IP
(86) International application number: PCT/EP2022/083079
(87) International publication number: WO 2023/094502

(56) References cited:
- TOBAL IGNACIO E. ET AL: "1,3-Cyclohexadien-1-Als: Synthesis, Reactivity and Bioactivities", vol. 26, no. 6, 1 January 2021 (2021-01-01), pages 1772, XP055917383, Retrieved from the Internet <URL:https://www.ncbi.nlm.nih.gov/pmc/articles/PMC8004145/pdf/molecules-26-01772.pdf> DOI: 10.3390/molecules26061772

## Description

### Technical field

The present invention relates to the field of organic synthesis and more specifically it concerns a process for preparing compound of formula (I) comprising the reaction of a compound of the formula (II) with a base having a boiling point superior to the boiling point of the compound of formula (I). Moreover, the compound of formula (I) and the compound of formula (II) are also part of the invention.

### Background

The conjugated cyclic derivatives represent skeletons highly desirables which could be used as such or as key intermediates useful to prepare more complex compounds in different fields such as, among others, perfumery, cosmetic, pharmaceutic or agrochemistry. Relevant conjugated cyclic derivatives in perfumery industry are, for example, 1-(2,6,6-trimethyl-1-cyclohexa-1,3-dienyl)but-2-en-1-one or ethyl 2,6,6-trimethylcyclohexa-1,3-diene-1-carboxylate, particularly appreciated for their floral odor. Those perfuming ingredients represent some of the most sought-after ingredients in the perfumery industry. The methods known in the art to prepare those compounds, such as elimination step, provide a mixture of several regioisomers, also known as alpha, beta and gamma isomers, impossible to separate.

So, there is still a need to develop a highly selective method, preferably catalytic, affording conjugated cyclic derivatives with high yield.

Tobal et al (Molecules 2021, Vol. 26, nr. 6, p. 1772) described the preparation of a 1,3 cyclohexadiene n the presence of p-toluenesulfonic acid.

The present invention allows to solve the above problem by using a base having a boiling point superior to the boiling point of the compound of formula (I) in order to prepare a conjugated dienes of formula (I); i.e. beta isomer. To the best of our knowledge, the invention's conditions have never been reported in the prior art.

### Summary of the Invention

The invention relates to a novel process allowing the preparation of compound of formula (I), while limiting even preventing the formation of others regioisomers, using methodologies never reported or suggested in the prior art.

So, a first object of the present invention is a process for the preparation of a compound of formula (I) 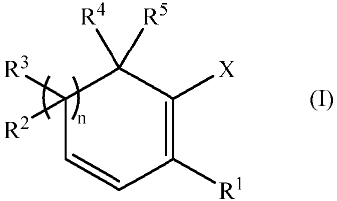 in a form of any one of its stereoisomers or a mixture thereof and wherein n is an integer comprised between 0 and 13; R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group; comprising the reaction of a compound of the formula (II) 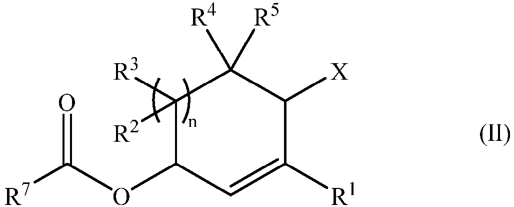 in a form of any one of its stereoisomers and wherein n, X, R¹, R², R³, R⁴ and R⁵ have the same meaning as defined in formula (I) and R⁷ represents a hydrogen atom, a C₂₋₆ alkenyl group, a C₁₋₆ alkyl group optionally substituted by one or more of a halogen atom, or a C₆₋₁₀ aryl group, optionally substituted by one or more of a hydroxy, C₁₋₆ alkyl, C₂₋₆ alkenyl, C₁₋₆ alkoxy group and/or halogen atom;
with a base having a boiling point superior to the boiling point of the compound of formula (I).

Another object of the present invention is a compound of formula 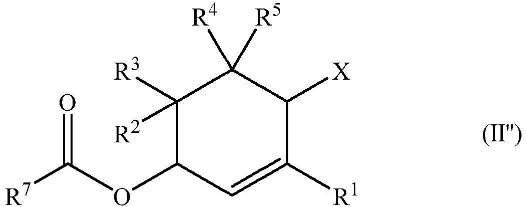 in a form of any one of its stereoisomers and wherein R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group and R⁷ represents a hydrogen atom, a C₂₋₆ alkenyl group, a C₁₋₆ alkyl group optionally substituted by one or more of a halogen atom, or a C₆₋₁₀ aryl group, optionally substituted by one or more of a hydroxy, C₁₋₆ alkyl, C₂₋₆ alkenyl, C₁₋₆ alkoxy group and/or halogen atom; provided that 4-(but-2-enoyl)-3,5,5-trimethylcyclohex-2-en-1-yl acetate and ethyl 4-acetoxy-2,6,6-trimethylcyclohex-2-ene-1-carboxylate are excluded.

A further object of the present invention is a compound of formula 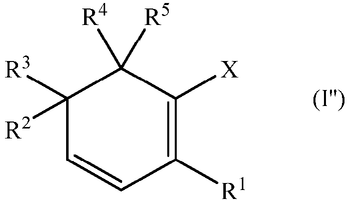 in a form of any one of its stereoisomers or a mixture thereof and wherein R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group; provided that ethyl 2-ethyl-6,6-dimethylcyclohexa-1,3-diene-1-carboxylate, methyl 2,6,6-trimethylcyclohexa-1,3-diene-1-carboxylate, ethyl 2,6,6-trimethylcyclohexa-1,3-diene-1-carboxylate, 1-(2,6,6-trimethylcyclohexa-1,3-dien-1-yl)ethan-1-one and 1-(2,6,6-trimethylcyclohexa-1,3-dien-1-yl)but-2-en-1-one are excluded.

### Description of the invention

Surprisingly, it has now been discovered that the compound of formula (I) can be produced in an advantageous manner by reacting compound of formula (II) with a base having a boiling point superior to the boiling point of the compound of formula (I). This unprecedented step allows the generation of compound of formula (I) with a high selectivity toward the beta isomer, limiting even avoiding the formation of the alpha and/or gamma isomers.

Therefore, a first object of the present invention is a process for the preparation of a compound of formula (I) 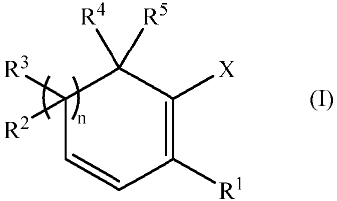 in a form of any one of its stereoisomers or a mixture thereof and wherein n is an integer comprised between 0 and 13; R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group; comprising the reaction of a compound of the formula (II) 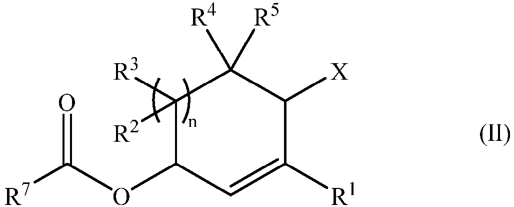 in a form of any one of its stereoisomers and wherein n, X, R¹, R², R³, R⁴ and R⁵ have the same meaning as defined in formula (I) and R⁷ represents a hydrogen atom, a C₂₋₆ alkenyl group, a C₁₋₆ alkyl group optionally substituted by one or more of a halogen atom, or a C₆₋₁₀ aryl group, optionally substituted by one or more of a hydroxy, C₁₋₆ alkyl, C₂₋₆ alkenyl, C₁₋₆ alkoxy group and/or halogen atom;
with a base having a boiling point superior to the boiling point of the compound of formula (I).

For the sake of clarity, by the expression "any one of its stereoisomers or a mixture thereof", or the similar, it is meant the normal meaning understood by a person skilled in the art, i.e. that the compounds cited in the invention can be a pure enantiomer or a mixture of enantiomers. In other words, the compounds cited in the invention may possess at least one stereocenter which can have two different stereochemistries (e.g. R or S), e.g. the R¹ group may comprise at least one stereocenter. Said compounds may even be in the form of a pure enantiomer or in the form of a mixture of enantiomers. The compounds cited in the invention may even be in the form of a pure diastereoisomer or in the form of a mixture of diastereoisomers when said compounds possess more than one stereocenter or at least one double bond. Said compounds can be in a racemic form or scalemic form. Therefore, said compounds can be one stereoisomer or in the form of a composition of matter comprising, or consisting of, various stereoisomers.

According to any one of the above embodiments of the invention, said compounds can be in the form of its E or Z isomer or of a mixture thereof, e.g. the invention comprises compositions of matter consisting of one or more compounds of formula (I) and (II), having the same chemical structure but differing by the configuration of the double bond. In particular, compound (I) and compound (II) can be in the form of a mixture consisting of isomers E and Z.

The term "optionally" is understood that a group can or cannot be substituted. The term "one or more" is understood as comprising 1 to 7, preferably, 1 to 5, preferably, 1 to 3 functional groups and more preferably 1 or 2 functional groups.

The terms "alkyl" and "alkenyl" are understood as comprising branched and linear alkyl and alkenyl groups.

The term "alkenyl" is understood as comprising 1, 2 or 3 olefinic double bonds, preferably 1 or 2 olefinic double bonds.

The term "C₃₋₆ cycloalkyl" is understood as a saturated ring comprising from 3 to 6 carbon atoms and including the carbon atom to which said R⁴ and R⁵ groups are bonded.

The term "aryl" is understood as comprising any group comprising at least one aromatic group such as phenyl, indenyl, indanyl, tetrahydronaphthalenyl or naphthalenyl group.

The term "CN group" is understood as a nitril group; i.e. -C≡N group.

According to any embodiment of the invention, n may be an integer between 0 and 10, particularly between 0 and 8, particularly between 0 and 6, particularly between 0 and 4. Particularly, n may be 0, 1 or 2. Even More particularly, n may be 1.

According to any embodiment of the invention, R² may be a hydrogen atom, a C₁₋₄ alkyl group or a C₂₋₄ alkenyl group. Particularly, R² may be a hydrogen atom or a C₁₋₃ alkyl group. R² may be a hydrogen atom, a methyl or an ethyl group. Even more particularly, R² may be a hydrogen atom.

According to any embodiment of the invention, R³ may be a hydrogen atom, a C₁₋₄ alkyl group or a C₂₋₄ alkenyl group. Particularly, R³ may be a hydrogen atom or a C₁₋₃ alkyl group. R³ may be a hydrogen atom, a methyl or an ethyl group. Even more particularly, R³ may be a hydrogen atom.

According to any embodiment of the invention, the compound of formula (I) is a compound of formula 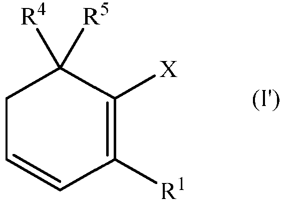 in a form of any one of its stereoisomers or a mixture thereof and wherein n, R¹, R⁴ and R⁵ have the same meaning as defined above.

According to any embodiment of the invention, the compound of formula (II) is a compound of formula 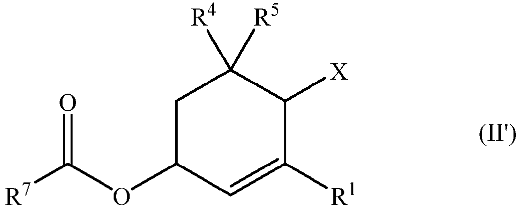 in a form of any one of its stereoisomers and wherein n, X, R¹, R⁴, R⁵ and R⁷ have the same meaning as defined above.

According to any embodiment of the invention, R¹ may be a hydrogen atom, a C₁₋₄ alkyl group or a C₂₋₄ alkenyl group. Particularly, R¹ may be a hydrogen atom or a C₁₋₃ alkyl group. R¹ may be a hydrogen atom, a methyl or an ethyl group. Even more particularly, R¹ may be a methyl or an ethyl group.

According to any embodiment of the invention, R⁴ may be a hydrogen atom, a C₁₋₄ alkyl group or a C₂₋₄ alkenyl group. Particularly, R⁴ may be a hydrogen atom or a C₁₋₃ alkyl group. R⁴ may be a hydrogen atom, a methyl or an ethyl group. Even more particularly, R⁴ may be a methyl or an ethyl group.

According to any embodiment of the invention, R⁵ may be a hydrogen atom, a C₁₋₄ alkyl group or a C₂₋₄ alkenyl group. Particularly, R⁵ may be a hydrogen atom or a C₁₋₃ alkyl group. R⁵ may be a hydrogen atom, a methyl or an ethyl group. Even more particularly, R⁵ may be a methyl or an ethyl group.

According to any embodiment of the invention, R⁴ and R⁵ may be taken together and form a C₃₋₅ cycloalkyl group. Particularly, R⁴ and R⁵ may be taken together and form a C₃ cycloalkyl group.

According to any embodiment of the invention, R⁶ may be a hydrogen atom, a C₁₋₄ alkyl group or a C₂₋₄ alkenyl group. Particularly, R⁶ may be a hydrogen atom, a C₁₋₃ alkyl group or a C₂₋₃ alkenyl group. R⁶ may be a hydrogen atom, a methyl, an ethyl or a prop-1-en-1-yl group. Even more particularly, R⁶ may be a methyl or an ethyl group.

According to any embodiment of the invention, X may be a C(O)R⁶ or COOR⁶ group wherein R⁶ has the same meaning as defined above.

According to any embodiment of the invention, R⁷ may be a hydrogen atom, a C₂₋₄ alkenyl group, a C₁₋₄ alkyl group optionally substituted by one, two or three halogen atoms, or a phenyl group optionally substituted by one or more of a hydroxy, C₁₋₆ alkyl, C₂₋₆ alkenyl, C₁₋₆ alkoxy group and/or halogen atom. Particularly, R⁷ may be a hydrogen atom, a C₂₋₄ alkenyl group, a C₁₋₄ alkyl group optionally substituted by one, two or three halogen atoms, or a phenyl group optionally substituted by one or more of a hydroxy, C₁₋₄ alkyl, C₂₋₄ alkenyl, C₁₋₄ alkoxy group and/or halogen atom. Particularly, R⁷ may be a hydrogen atom, a C₂₋₃ alkenyl group, a C₁₋₃ alkyl group optionally substituted by one, two or three halogen atoms, or a phenyl group, optionally substituted by one or more of a hydroxy, C₁₋₃ alkyl, C₂₋₃ alkenyl, C₁₋₃ alkoxy group and/or halogen atom. Particularly, R⁷ may be a methyl, ethyl, propyl, isopropyl or phenyl group. Even more particularly, R⁷ may be a methyl group.

Non-limiting examples of suitable compounds of formula (I) may include 1-(2,6,6-trimethylcyclohexa-1,3-dien-1-yl)but-2-en-1-one, 2-methyl-1-(2,6,6-trimethylcyclohexa-1,3-dien-1-yl)but-2-en-1-one, 1-(6-ethyl-2,6-dimethylcyclohexa-1,3-dien-1-yl)-2-methylbut-2-en-1-one, 1-(6-ethyl-2,6-dimethylcyclohexa-1,3-dien-1-yl)but-2-en-1-one, 1-(5-methylspiro[2.5]octa-4,6-dien-4-yl)but-2-en-1-one, 1-(5-methylspiro[2.5]octa-4,6-dien-4-yl)ethan-1-one, 1-(2,6,6-trimethylcyclohexa-1,3-dien-1-yl)ethan-1-one, 1-(6-ethyl-2,6-dimethylcyclohexa-1,3-dien-1-yl)ethan-1-one, methyl or ethyl 6-ethyl-2,6-dimethylcyclohexa-1,3-diene-1-carboxylate, methyl or ethyl 2,6,6-trimethylcyclohexa-1,3-diene-1-carboxylate, 1-(2,5,6-trimethylcyclohexa-1,3-dien-1-yl)but-2-en-1-one, methyl or ethyl 2-ethyl-6,6-dimethylcyclohexa-1,3-diene-1-carboxylate, methyl or ethyl 2,5,6-trimethylcyclohexa-1,3-diene-1-carboxylate, methyl or ethyl 5-methylspiro[2.5]octa-4,6-diene-4-carboxylate.

Non-limiting examples of suitable compounds of formula (II) may include 4-acetyl-3,5,5-trimethylcyclohex-2-en-1-yl acetate, 4-(but-2-enoyl)-3,5,5-trimethylcyclohex-2-en-1-yl acetate, 3,5,5-trimethyl-4-(2-methylbut-2-enoyl)cyclohex-2-en-1-yl acetate, 5-ethyl-3,5-dimethyl-4-(2-methylbut-2-enoyl)cyclohex-2-en-1-yl acetate, 4-(but-2-enoyl)-5-ethyl-3,5-dimethylcyclohex-2-en-1-yl acetate, 8-(but-2-enoyl)-7-methylspiro[2.5]oct-6-en-5-yl acetate, 4-acetyl-3,5,5-trimethylcyclohex-2-en-1-yl acetate, 4-acetyl-5-ethyl-3,5-dimethylcyclohex-2-en-1-yl acetate, 8-acetyl-7-methylspiro[2.5]oct-6-en-5-yl acetate, methyl or ethyl 7-acetoxy-5-methylspiro[2.5]oct-5-ene-4-carboxylate, methyl or ethyl 4-acetoxy-2,6,6-trimethylcyclohex-2-ene-1-carboxylate, methyl or ethyl 4-acetoxy-6-ethyl-2,6-dimethylcyclohex-2-ene-1-carboxylate, methyl or ethyl 4-acetoxy-2,5,6-trimethylcyclohex-2-ene-1-carboxylate, 4-(but-2-enoyl)-3,5,5-trimethylcyclohex-2-en-1-yl acetate, 4-(but-2-enoyl)-3,5,6-trimethylcyclohex-2-en-1-yl acetate, methyl or ethyl 4-acetoxy-2-ethyl-6,6-dimethylcyclohex-2-ene-1-carboxylate, methyl or ethyl 7-acetoxy-5-methylspiro[2.5]oct-5-ene-4-carboxylate.

According to any embodiments of the invention, the base has a boiling point of at least 5°C greater than the boiling point of the compound of formula (I). Particularly, the base has a boiling point of at least 10°C, 15°C or even 20°C greater than the boiling point of the compound of formula (I).

According to any embodiments of the invention, the base is a metal carboxylate, a metal bicarbonate or a metal carbonate.

According to any embodiments of the invention, the metal carbonate is of formula

MₚCO₃ (III)

wherein M is a alkali metal or a alkaline earth metal and p is 1 or 2; provided that when M is an alkaline earth metal, then p is 1 and when M is a alkali metal, then p is 2.

According to any embodiments of the invention, the metal bicarbonate is of formula

M(HOCO₂)_{q} (IV)

wherein M is a alkali metal or a alkaline earth metal and q is 1 or 2; provided that when M is an alkaline earth metal, then q is 2 and when M is a alkali metal, then q is 1.

According to any embodiments of the invention, the metal carboxylate is of formula

(RCOO)ᵣM (V)

wherein R is a hydrogen atom or a C₁₋₁₈ hydrocarbon group; M is a alkali metal or a alkaline earth metal and r is 1 or 2; provided that when M is an alkali metal then r is 1 and when M is an alkaline earth metal then r is 2.

It is understood that by "... hydrocarbon group ..." it is meant that said group consists of hydrogen and carbon atoms and can be in the form of an aliphatic hydrocarbon, i.e. linear or branched saturated hydrocarbon (e.g. alkyl group), a linear or branched unsaturated hydrocarbon (e.g. alkenyl or alkynyl group), a saturated cyclic hydrocarbon (e.g. cycloalkyl) or an unsaturated cyclic hydrocarbon (e.g. cycloalkenyl or cycloalkynyl), or can be in the form of an aromatic hydrocarbon, i.e. aryl group, or can also be in the form of a mixture of said type of groups, e.g. a specific group may comprise a linear alkyl, a branched alkenyl (e.g. having one or more carbon-carbon double bonds), a (poly)cycloalkyl and an aryl moiety, unless a specific limitation to only one type is mentioned. Similarly, in all the embodiments of the invention, when a group is mentioned as being in the form of more than one type of topology (e.g. linear, cyclic or branched) and/or being saturated or unsaturated (e.g. alkyl, aromatic or alkenyl), it is also meant a group which may comprise moieties having any one of said topologies or being saturated or unsaturated, as explained above. Similarly, in all the embodiments of the invention, when a group is mentioned as being in the form of one type of saturation or unsaturation, (e.g. alkyl), it is meant that said group can be in any type of topology (e.g. linear, cyclic or branched) or having several moieties with various topologies.

According to any embodiments of the invention, M may be selected from the group of magnesium, calcium, potassium, sodium and lithium, preferably potassium.

According to any embodiments of the invention, the invention's process is performed in the presence of a metal carboxylate.

According to any embodiments of the invention, R may be a hydrogen atom or a C₁₋₁₅ hydrocarbon group. Particularly, R may be a hydrogen atom, a phenyl group or a C₁₋₁₅ alkyl group or C₂₋₁₅ alkenyl group. Particularly, R may be a hydrogen atom, a phenyl group or a C₁₋₁₀ alkyl group or C₂₋₁₀ alkenyl group. Particularly, R may be a hydrogen atom, a phenyl group or a C₁₋₈ alkyl group or C₂₋₈ alkenyl group. Particularly, R may be a hydrogen atom, a phenyl group or a C₁₋₆ alkyl group. Particularly, R may be a hydrogen atom, a phenyl group or a C₁₋₃ alkyl group. Even more particularly, R may be a methyl group.

Non-limiting examples of suitable of metal carbonate may include potassium carbonate, magnesium carbonate, calcium carbonate, sodium carbonate or lithium carbonate.

Non-limiting examples of suitable of metal bicarbonate may include potassium bicarbonate, magnesium bicarbonate, calcium bicarbonate, sodium bicarbonate or lithium bicarbonate

Non-limiting examples of suitable of metal carboxylate may include potassium acetate, magnesium acetate, sodium acetate, calcium acetate, lithium acetate, potassium benzoate, magnesium benzoate, sodium benzoate, calcium benzoate, lithium benzoate potassium propionate, magnesium propionate, sodium propionate, calcium propionate, lithium propionate, potassium octoate, magnesium octoate, sodium octoate, calcium octoate, lithium octoate, potassium 2-ethylhexanoate, magnesium 2-ethylhexanoate, sodium 2-ethylhexanoate, calcium 2-ethylhexanoate or lithium 2-ethylhexanoate.

The metal bicarbonate or carbonate can be added into the reaction medium of the invention's process to form compound of formula (I) in a large range of concentrations. As non-limiting examples, one can cite, as metal bicarbonate or carbonate concentration values those ranging from 1 mol% to 10 equivalents, relative to the total amount of compound of formula (II). Particularly, the metal bicarbonate or carbonate concentration may be comprised between 1 mol% to 5 equivalents. Particularly, the metal bicarbonate or carbonate concentration may be comprised between 1.2 mol% to 2 equivalents. It goes without saying that the process works also with more metal bicarbonate or carbonate. However the optimum concentration of metal bicarbonate or carbonate will depend, as the person skilled in the art knows, on the nature of the latter, on the nature of the compound of formula (II), on the temperature and on the desired time of reaction.

According to any embodiments of the invention, the invention's process is carried out in the presence of a catalytic amount of the metal carboxylate.

The metal carboxylate can be added into the reaction medium of the invention's process to form compound of formula (I) in a large range of concentrations. As non-limiting examples, one can cite, as metal carboxylate concentration values those ranging from 0.1 mol% to 100 mol%, relative to the total amount of compound of formula (II). Particularly, the metal carboxylate concentration may be comprised between 1 mol% to 50 mol%. Particularly, the metal carboxylate concentration may be comprised between 2 mol% to 20 mol%. Even more particularly, the metal carboxylate concentration may be comprised between 5 mol% to 15 mol%. It goes without saying that the process works also with more metal carboxylate. However the optimum concentration of metal carboxylate will depend, as the person skilled in the art knows, on the nature of the latter, on the nature of the compound of formula (II), on the temperature and on the desired time of reaction.

The invention's process to form compound of formula (I) is carried out under batch, semi continuous or continuous conditions.

According to any embodiments of the invention, the more volatile compounds generated during the invention's process such as the compound of formula (I) and, optionally the carboxylic acid of formula R⁷COOH, are removed continuously during the process, for example, by distillation at atmospheric pressure or under reduced pressure.

The invention's process may be performed under inert atmosphere such as nitrogen or argon. The invention's process may be performed under atmospheric pressure or under a slight vacuum. Particularly, the invention's process can be carried out at a pressure comprised between 100 Pa and 10000 Pa (1 to 100 mbars), particularly, between 3000 Pa and 5000 Pa (30 to 50 mbars). A person skilled in the art is well able to adjust the pressure as a function of the compound of formula (II) and (I) and the solvent.

The invention's process to form compound of formula (I) can be carried out in the presence or absence of a solvent. When a solvent is required or used for practical reasons, then any solvent current in such reaction type can be used for the purposes of the invention, particularly at least one aprotic dipolar solvent. Non-limiting examples include 2-pyrrolidone, N-Methylpyrrolidone, N-octylpyrrolidone, dimethylformamide, dimethylacetamide, dimethylsulfoxide, sulfolane, hexamethyl phosphoramide or a mixture thereof. The choice of the solvent is function of the nature of the compound of formula (II) and/or the base and the person skilled in the art is well able to select the solvent most suitable in each case to optimize the reaction.

According to any one of the invention's embodiments, the invention's process to form compound of formula (I) is carried out at a temperature comprised between 80°C and 250°C. In particular, the temperature is in the range between 100°C and 200°C. A person skilled in the art is also able to select the preferred temperature as a function of the melting and boiling point of the starting and final products as well as the desired time of reaction or conversion.

The compound of formula (I), and (II) are, generally, novel compounds and present a number of advantages as explained above and shown in the Examples.

Therefore, another object of the present invention is a compound of formula 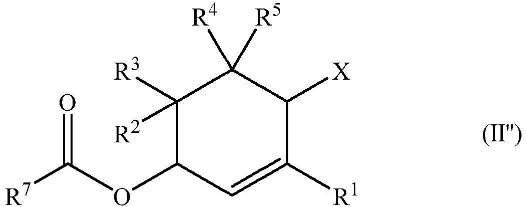 in a form of any one of its stereoisomers and wherein R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group and R⁷ represents a hydrogen atom, a C₂₋₆ alkenyl group, a C₁₋₆ alkyl group optionally substituted by one or more of a halogen atom, or a C₆₋₁₀ aryl group, optionally substituted by one or more of a hydroxy, C₁₋₆ alkyl, C₂₋₆ alkenyl, C₁₋₆ alkoxy group and/or halogen atom; provided that 4-(but-2-enoyl)-3,5,5-trimethylcyclohex-2-en-1-yl acetate and ethyl 4-acetoxy-2,6,6-trimethylcyclohex-2-ene-1-carboxylate are excluded.

According to any embodiment of the invention, the compound of formula (II) may be selected in the group consisting of 4-acetyl-3,5,5-trimethylcyclohex-2-en-1-yl acetate, 3,5,5-trimethyl-4-(2-methylbut-2-enoyl)cyclohex-2-en-1-yl acetate, 5-ethyl-3,5-dimethyl-4-(2-methylbut-2-enoyl)cyclohex-2-en-1-yl acetate, 4-(but-2-enoyl)-5-ethyl-3,5-dimethylcyclohex-2-en-1-yl acetate, 8-(but-2-enoyl)-7-methylspiro[2.5]oct-6-en-5-yl acetate, 4-acetyl-3,5,5-trimethylcyclohex-2-en-1-yl acetate, 4-acetyl-5-ethyl-3,5-dimethylcyclohex-2-en-1-yl acetate, 8-acetyl-7-methylspiro[2.5]oct-6-en-5-yl acetate, methyl 7-acetoxy-5-methylspiro[2.5]oct-5-ene-4-carboxylate, ethyl 7-acetoxy-5-methylspiro[2.5]oct-5-ene-4-carboxylate, methyl 4-acetoxy-2,6,6-trimethylcyclohex-2-ene-1-carboxylate, methyl 4-acetoxy-6-ethyl-2,6-dimethylcyclohex-2-ene-1-carboxylate, ethyl 4-acetoxy-6-ethyl-2,6-dimethylcyclohex-2-ene-1-carboxylate, 4-(but-2-enoyl)-3,5,6-trimethylcyclohex-2-en-1-yl acetate, methyl 4-acetoxy-2-ethyl-6,6-dimethylcyclohex-2-ene-1-carboxylate, ethyl 4-acetoxy-2-ethyl-6,6-dimethylcyclohex-2-ene-1-carboxylate, methyl 4-acetoxy-2,5,6-trimethylcyclohex-2-ene-1-carboxylate, ethyl 4-acetoxy-2,5,6-trimethylcyclohex-2-ene-1-carboxylate, methyl 7-acetoxy-5-methylspiro[2.5]oct-5-ene-4-carboxylate and ethyl 7-acetoxy-5-methylspiro[2.5]oct-5-ene-4-carboxylate.

Another object of the present invention is a compound of formula 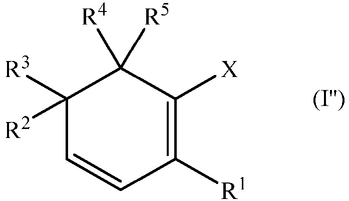 in a form of any one of its stereoisomers or a mixture thereof and wherein R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group; provided that ethyl 2-ethyl-6,6-dimethylcyclohexa-1,3-diene-1-carboxylate, methyl 2,6,6-trimethylcyclohexa-1,3-diene-1-carboxylate, ethyl 2,6,6-trimethylcyclohexa-1,3-diene-1-carboxylate, 1-(2,6,6-trimethylcyclohexa-1,3-dien-1-yl)ethan-1-one and 1-(2,6,6-trimethylcyclohexa-1,3-dien-1-yl)but-2-en-1-one are excluded.

According to any embodiment of the invention, the compound of formula (I) may be selected in the group consisting of 2-methyl-1-(2,6,6-trimethylcyclohexa-1,3-dien-1-yl)but-2-en-1-one, 1-(6-ethyl-2,6-dimethylcyclohexa-1,3-dien-1-yl)-2-methylbut-2-en-1-one, 1-(6-ethyl-2,6-dimethylcyclohexa-1,3-dien-1-yl)but-2-en-1-one, 1-(5-methylspiro[2.5]octa-4,6-dien-4-yl)but-2-en-1-one, 1-(5-methylspiro[2.5]octa-4,6-dien-4-yl)ethan-1-one, 1-(6-ethyl-2,6-dimethylcyclohexa-1,3-dien-1-yl)ethan-1-one, methyl 6-ethyl-2,6-dimethylcyclohexa-1,3-diene-1-carboxylate, ethyl 6-ethyl-2,6-dimethylcyclohexa-1,3-diene-1-carboxylate, 1-(2,5,6-trimethylcyclohexa-1,3-dien-1-yl)but-2-en-1-one, methyl 2-ethyl-6,6-dimethylcyclohexa-1,3-diene-1-carboxylate, ethyl 5-methylspiro[2.5]octa-4,6-diene-4-carboxylate, methyl 5-methylspiro[2.5]octa-4,6-diene-4-carboxylate, methyl 2,5,6-trimethylcyclohexa-1,3-diene-1-carboxylate, and ethyl 2,5,6-trimethylcyclohexa-1,3-diene-1-carboxylate.

Said compound of formula (I) can be used as perfuming ingredient, for instance to impart odor notes of the amber type. So another object of the invention is the use as perfuming ingredient of the invention's compound of formula (I) as defined above. In other words, it concerns a method or a process to confer, enhance, improve or modify the odor properties of a perfuming composition or of a perfumed article or of a surface, which method comprises adding to said composition or article an effective amount of the invention's composition of matter, e.g. to impart its typical note. Understood that the final hedonic effect may depend on the precise dosage and on the organoleptic properties of the invention's composition of matter, but anyway the addition of the invention's compound of formula (I) will impart to the final product its typical touch in the form of a note, touch or aspect depending on the dosage.

By "use of the the invention's compound of formula (I)" it has to be understood here also the use of any composition containing the invention's compound of formula (I) and which can be advantageously employed in the perfumery industry.

Said compositions, which in fact can be advantageously employed as perfuming ingredients, are also an object of the present invention.

Therefore, another object of the present invention is a perfuming composition comprising:
i) as a perfuming ingredient, at least one compound of formula (I) as defined above;
ii) at least one ingredient selected from the group consisting of a perfumery carrier and a perfumery base; and
iii) optionally at least one perfumery adjuvant.

By "perfumery carrier" it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, Abalyn^{®} (rosin resins, available from Eastman), benzyl benzoate, benzyl alcohol, 2-(2-ethoxyethoxy)-1-ethanol, tri-ethyl citrate or mixtures thereof, which are the most commonly used or also naturally derived solvents like glycerol or various vegetable oils such as palm oil, sunflower oil or linseed oil. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. Solid carriers are of current use in the art and a person skilled in the art knows how to reach the desired effect. However by way of non-limiting examples of solid carriers, one may cite absorbing gums or polymers or inorganic materials, such as porous polymers, cyclodextrins, dextrins, maltodextrines wood based materials, organic or inorganic gels, clays, gypsum talc or zeolites.

As other non-limiting examples of solid carriers, one may cite encapsulating materials. Examples of such materials may comprise wall-forming and plasticizing materials, such as glucose syrups, natural or modified starches, hydrocolloids, cellulose derivatives, polyvinyl acetates, polyvinylalcohols, proteins or pectins, plant gums such as acacia gum (Gum Arabic), urea, sodium chloride, sodium sulfate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, carbohydrates, saccharides such as sucrose, mono-, di-, tri- and polysaccharides and derivatives such as chitosan, starch, cellulose, carboxymethyl methylcellulose, methylcellulose, hydroxyethyl cellulose, ethyl cellulose, propyl cellulose, polyols/sugar alcohols such as sorbitol, maltitol, xylitol, erythritol, and isomalt, polyethylene glycol (PEG), polyvinyl pyrrolidin (PVP), polyvinyl alcohol, acrylamides, acrylates, polyacrylic acid and related structures, maleic anhydride copolymers, amine-functional polymers, vinyl ethers, styrenes, polystyrenesulfonates, vinyl acids, ethylene glycol-propylene glycol block copolymers, vegetable gums, gum acacia, pectins, xanthanes, alginates, carragenans, citric acid or any water soluble solid acid, fatty alcohols or fatty acids and mixtures thereof, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture threof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycouryl, melamine, methylol melamine, methylated methylol melamine, guanazole and the like, as well as mixtures thereof. Alternatively, one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Other resins are the ones produced by the polycondensation of a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate are preferred.

Some of the seminal literature related to the encapsulation of perfumes by polycondensation of amino resins, namely melamine based resins with aldehydes includes articles such as those published by K. Dietrich et al. Acta Polymerica, 1989, Vol. 40, pages 243, 325 and 683, as well as 1990, Vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinence, which disclose suitable uses of such microcapsules, are represented for example by the article of K. Bruyninckx and M. Dusselier, ACS Sustainable Chemistry & Engineering, 2019, vol. 7, pages 8041-8054.

By "perfumery base" what is meant here is a composition comprising at least one perfuming co-ingredient.

Said perfuming co-ingredient is not compound of formula (I). Moreover, by "perfuming co-ingredient" it is meant here a compound, which is used in a perfuming preparation or a composition to impart a hedonic effect; i.e. used for the primary purpose of conferring or modulating an odor. In other words such a co-ingredient, to be considered as being a perfuming one, must be recognized by a person skilled in the art as being able to impart or modify in a positive or pleasant way the odor of a composition, and not just as having an odor. The perfuming co-ingredient may impart an additional benefit beyond that of modifying or imparting an odor, such as long-lasting, blooming, malodour counteraction, antimicrobial effect, antiviral effect, microbial stability, or pest control.

The nature and type of the perfuming co-ingredients present in the base do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, thiols, terpene hydrocarbons, nitrogenous or sulfurous heterocyclic compounds and essential oils, and said perfuming co-ingredients can be of natural or synthetic origin.

In particular one may cite perfuming co-ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0~2,7~]undecan-4-one, 1-methoxy-3-hexanethiol, 2-ethyl-4,4-dimethyl-1,3-oxathiane, 2,2,7/8,9/10-Tetramethylspiro[5.5]undec-8-en-1-one, menthol and/or alpha-pinene;
- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients: methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-1-carboxylate, 3-(4,4-dimethyl-1-cyclohexen-1-yl)propanal, 3-(3,3/1,1-dimethyl-5-indanyl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{(1S)-1-[(1R)-3,3-dimethylcyclohexyl]ethoxy}-2-oxoethyl propionate, 3-methyl-5-cyclopentadecen-1-one, 4,6,6,7,8,8-hexamethyl-1,3,4,6,7,8-hexahydrocyclopenta[g]isochromene, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®} (Origin: Firmenich SA), (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonane and/or 3-(3-isopropyl-1-phenyl)butanal.

A perfumery base according to the invention may not be limited to the above-mentioned perfuming co-ingredients, and many other of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfume or profragrance. Non-limiting examples of suitable properfume may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 3-(dodecylsulfonyl)-1-(2,6,6-trimethylcyclohex-3-en-1-yl)butan-1-one, a linear polysiloxane co-polymer of (3-mercaptopropyl)(methyl)dimethoxysilane, 1-[6-ethyl-2,6-dimethyl-3-cyclohexen-1-yl]-2-buten-1-one, 2-(dodecylthio)octan-4-one, 2-(dodecylsulfonyl)octan-4-one, 4-oxooctan-2-yl dodecanoate, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2E,6Z)-2,6-nonadienyl hexadecanoate, (2E,6Z)-2,6-nonadien-1-yl tetradecanoate, (2E,6Z)-2,6-nonadien-1-yl dodecanoate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-methoxy-4-(1-phenethoxyprop-1-en-2-yl)benzene, (2-((2-methyl-4-(2,6,6-trimethylcyclohex-1-en-1-yl)but-1-en-1-yl)oxy)ethyl)benzene, 1-methoxy-4-(2-methyl-3-phenethoxyallyl)benzene, (2-((2-isopropyl-5-methylcyclohexylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 2-ethoxy-1-((2-methoxy-2-phenylvinyl)oxy)-4-methylbenzene, 3-methoxy-4-((2-methoxy-2-phenylvinyl)oxy)benzaldehyde, 1-isopropyl-2-((2-methoxy-2-phenylvinyl)oxy)-4-methylbenzene, 4-((2-(hexyloxy)-2-phenylvinyl)oxy)-3-methoxybenzaldehyde or a mixture thereof.

By "perfumery adjuvant", it is meant here an ingredient capable of imparting additional added benefit such as a color, a particular light resistance, chemical stability, etc. A detailed description of the nature and type of adjuvant commonly used in perfuming composition cannot be exhaustive, but it has to be mentioned that said ingredients are well known to a person skilled in the art. One may cite as specific non-limiting examples the following: viscosity agents (e.g. surfactants, thickeners, gelling and/or rheology modifiers), stabilizing agents (e.g. preservatives, antioxidant, heat/light and or buffers or chelating agents, such as BHT), coloring agents (e.g. dyes and/or pigments), preservatives (e.g. antibacterial or antimicrobial or antifungal or anti irritant agents), abrasives, skin cooling agents, fixatives, insect repellants, ointments, vitamins and mixtures thereof. By "fixative" also called "modulator", it is understood here an agent having the capacity to affect the manner in which the odour, and in particular the evaporation rate and intensity, of the compositions incorporating said modulator can be perceived by an observer or user thereof, over time, as compared to the same perception in the absence of the modulator. In particular, the modulator allows prolonging the time during which their fragrance is perceived. Non-limiting examples of suitable modulators may include methyl glucoside polyol; ethyl glucoside polyol; propyl glucoside polyol; isocetyl alcohol; PPG-3 myristyl ether; neopentyl glycol diethylhexanoate; sucrose laurate; sucrose dilaurate, sucrose myristate, sucrose palmitate, sucrose stearate, sucrose distearate, sucrose tristearate, hyaluronic acid disaccharide sodium salt, sodium hyaluronate, propylene glycol propyl ether; dicetyl ether; polyglycerin-4 ethers; isoceteth-5; isoceteth-7, isoceteth-10; isoceteth-12; isoceteth-15; isoceteth-20; isoceteth-25; isoceteth-30; disodium lauroamphodipropionate; hexaethylene glycol monododecyl ether; and their mixtures; neopentyl glycol diisononanoate; cetearyl ethylhexanoate; panthenol ethyl ether, DL-panthenol, N-hexadecyl n-nonanoate, noctadecyl n-nonanoate, a profragrance, cyclodextrin, an encapsulation, and a combination thereof. At most 20% by weight, based on the total weight of the perfuming composition, of the modulator may be incorporated into the perfumed consumer product.

It is understood that a person skilled in the art is perfectly able to design optimal formulations for the desired effect by admixing the above mentioned components of a perfuming composition, simply by applying the standard knowledge of the art as well as by trial and error methodologies.

An invention's composition consisting of the invention's compound of formula (I) and at least one perfumery carrier consists of a particular embodiment of the invention as well as a perfuming composition comprising the invention's composition of matter, at least one perfumery carrier, at least one perfumery base, and optionally at least one perfumery adjuvant.

For the sake of clarity, it is also understood that any mixture resulting directly from a chemical synthesis, e.g. a reaction medium without an adequate purification, in which the invention's compound of formula (I) would be involved as a starting, intermediate or endproduct could not be considered as a perfuming composition according to the invention as far as said mixture does not provide the inventive compound of formula (I) in a suitable form for perfumery. Thus, unpurified reaction mixtures are generally excluded from the present invention unless otherwise specified.

The invention's compound of formula (I) can also be advantageously used in all the fields of modern perfumery, i.e. fine or functional perfumery, to positively impart or modify the odor of a consumer product into which said invention's compound of formula (I) is added. Consequently, another object of the present invention consists of a perfumed consumer product comprising, as a perfuming ingredient, the invention's composition of matter, as defined above.

The invention's compound of formula (I) can be added as such or as part of an invention's perfuming composition.

For the sake of clarity, "perfumed consumer product" is meant to designate a consumer product which delivers at least a pleasant perfuming effect to the surface or space to which it is applied (e.g. skin, hair, textile, or home surface). In other words, a perfumed consumer product according to the invention is a perfumed consumer product which comprises a functional formulation, as well as optionally additional benefit agents, corresponding to the desired consumer product, and an olfactive effective amount of at least one invention's compound. For the sake of clarity, said perfumed consumer product is a non-edible product.

The nature and type of the constituents of the perfumed consumer product do not warrant a more detailed description here, which in any case would not be exhaustive, the skilled person being able to select them on the basis of his general knowledge and according to the nature and the desired effect of said product.

Non-limiting examples of suitable perfumed consumer products include a perfume, such as a fine perfume, a splash or eau de parfum, a cologne or a shave or after-shave lotion; a fabric care product, such as a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product; a body-care product, such as a hair care product (e.g. a shampoo, a leave-on or rinse-off hair conditioner, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care products); an air care product, such as an air freshener or a "ready to use" powdered air freshener which can be used in the home space (rooms, refrigerators, cupboards, shoes or car) and/or in a public space (halls, hotels, malls, etc..); or a home care product, such as a mold remover, a furnisher care product, a wipe, a dish detergent or a hard-surface (e.g. a floor, bath, sanitary or a window-cleaning) detergent; a leather care product; a car care product, such as a polish, a wax or a plastic cleaner.

Some of the above-mentioned perfumed consumer products may represent an aggressive medium for the invention's composition of matter, so that it may be necessary to protect the latter from premature decomposition, for example by encapsulation or by chemically binding it to another chemical which is suitable to release the invention's compound of formula (I) upon a suitable external stimulus, such as an enzyme, light, heat or a change of pH.

Typical manners to execute the invention's process are reported herein below in the examples.

### Examples

The invention will now be described in further detail by way of the following examples, wherein the abbreviations have the usual meaning in the art, the temperatures are indicated in degrees centigrade (°C). The solvents were dried by conventional procedures and distilled under an argon atmosphere. NMR spectra were recorded at 20 °C on Bruker AV 300, AV 400, or AV 500 MHz spectrometers. Chemical shifts are reported in ppm relative to solvent signals (chloroform, δ_{H} = 7.26 ppm, δ_{C} = 77.0 ppm). The signal assignment was ensured by recording ¹H,¹H- COSY, -NOESY, ¹³C,¹H-HSQC and -HMBC experiments. Gas chromatography was performed on an Agilent 7890 A Series equipped with a HP5 column (30 m x 0.25 mm ID, 0.25µm film) and tetradecane was used as internal standard.

### Example 1

### Preparation of 1-(2,6,6-trimethylcyclohexa-1,3-dien-1-yl)ethan-1-one following the invention's process

Into a 1L glass reactor equipped with a mechanical stirrer, a packed column, a reflux condenser, 60g (0.61 mol) of potassium acetate, 250g of 2-pyrrolidone and 250g (1.11 mol) of 4-acetyl-3,5,5-trimethylcyclohex-2-en-1-yl acetate were added. The reaction mixture was stirred and then heated to 135°C while gradually decreasing the internal pressure to 40 mbar. The formed product with acetic acid all together were continuously distilled into a flask over 4h. Then the reaction mixture was heated to 155°C while the pressure was decreased to 25 mbar. The distillate was taken up separately into another vessel to be neutralized by aqueous soda followed by washing with water. The subsequent fractionation of the resulting crude oil (188g) at 65-80°C/5 mbar afforded 169g of 1-(2,6,6-trimethylcyclohexa-1,3-dien-1-yl)ethan-1-one (92% yield). The analysis of the fraction by GC chromatography showed 97% of 1-(2,6,6-trimethylcyclohexa-1,3-dien-1-yl)ethan-1-one, 1.8% of 1-(6,6-dimethyl-2-methylenecyclohex-3-en-1-yl)ethan-1-one and 0.2% of 1-(2,6,6-trimethylcyclohexa-2,4-dien-1-yl)ethan-1-one.

H¹-NMR (CDCl₃, 500 MHz) δ 1.09 (s, 6H); 1.73 (s, 3H); 2.1 (dd, J1=4.1Hz, J2=1,5Hz, 2H); 2,3 (s, 3H); 5.81 (m, 2H).

13C-NMR (CDCl₃, 125MHz) δ 19,0 (q); 26.0 (2q); 33.1 (q); 33.7 (s); 39.8 (t); 126.2 (s); 127.4 (d); 128.1 (d); 142.3 (s); 208.3 (s).

### Example 2

### Preparation of ethyl 2,6,6-trimethylcyclohexa-1,3-diene-1-carboxylate following the invention's process

Into a 1L glass reactor equipped with a mechanical stirrer, a packed column, a reflux condenser, 50g (0.50 mol) of potassium acetate, 200g of 2-pyrrolidone and 300g (1.18 mol) of ethyl 4-acetoxy-2,6,6-trimethylcyclohex-2-ene-1-carboxylate were added. The reaction mixture was stirred then heated to 155°C while gradually decreasing the internal pressure to 50 mbar. The formed product with acetic acid all together were continuously distilled into a flask over 4h. Then the reaction mass was heated to 165°C while the pressure was decreased to 25 mbar. The distillate was taken up separately into another vessel to be neutralized by aqueous soda followed by washing with water. The subsequent fractionation of the resulting crude oil (228g) at 65-80°C/3 mbar afforded 199g of ethyl 2,6,6-trimethylcyclohexa-1,3-diene-1-carboxylate (88% yield). The analysis of the fraction by GC chromatography showed 97.2% of ethyl 2,6,6-trimethylcyclohexa-1,3-diene-1-carboxylate, 1.0% of ethyl 6,6-dimethyl-2-methylenecyclohex-3-ene-1-carboxylate and 1.0% of ethyl 2,6,6-trimethylcyclohexa-2,4-diene-1-carboxylate.

H¹-NMR (CDCl₃, 500 MHz) δ 1.12 (s, 6H); 1.33 (t, J = 7.0Hz, 3H); 1.81 (s, 3H); 2.1 (dd, J1=4.4Hz, J2=1,7Hz, 2H); 4.25 (q, J=7.0Hz, 2H); 5.81 (m, 1H); 5.87 (m, 1H).

13C-NMR (CDCl₃, 125MHz) δ 14.4 (q); 19.5 (q); 25.8 (2q); 33.3 (s); 39.4(t); 60.07 (t); 128.0 (d); 128.3 (d); 131.8 (s); 133.5 (s); 169.8(s).

### Example 3

### Preparation of methyl 2-ethyl-6,6-dimethylcyclohexa-1,3-diene-1-carboxylate following the invention's process

Into a 1L glass reactor equipped with a mechanical stirrer, a packed column, a reflux condenser, 50g (0.50 mol) of potassium acetate, 200g of 2-pyrrolidone and 300g (1.18 mol) of methyl 4-acetoxy-2-ethyl-6,6-dimethylcyclohex-2-ene-1-carboxylate were added. The reaction mixture was stirred and then heated to 155°C while gradually decreasing the internal pressure to 50 mbar. The formed product with acetic acid all together were continuously distilled into a flask over 4h. Then the reaction mass was heated to 165°C while the pressure was decreased to 25 mbar. The distillate was taken up separately into another vessel to be neutralized by aqueous soda followed by washing with water. The subsequent fractionation of the resulting crude oil (231g) at 65-80°C/3 mbar afforded 202g of the methyl 2-ethyl-6,6-dimethylcyclohexa-1,3-diene-1-carboxylate (89.5% yield). The analysis of the fraction by GC chromatography shows 98.5% of methyl 2-ethyl-6,6-dimethylcyclohexa-1,3-diene-1-carboxylate, 0.3% of methyl 2-ethylidene-6,6-dimethylcyclohex-3-ene-1-carboxylate and 0.9% of methyl 2-ethyl-6,6-dimethylcyclohexa-2,4-diene-1-carboxylate.

H¹-NMR (CDCl₃, 500 MHz) δ 1.01 (t, 3H); 1.10 (s, 6H); 2.08 (q, 2H); 2.11 (d, 2H); 3.75 (s, 3H); 5.87 (d, 1H); 5.89 (m, 1H).

¹³C-NMR (CDCl₃, 125MHz) δ 13.5; 25.9; 25.9; 27; 33.2; 39.4; 51; 126.4; 128.8; 132.7; 137.5; 170.3.

### Example 4

### Preparation of ethyl 2,5,6-trimethylcyclohexa-1,3-diene-1-carboxylate following the invention's process

Into a 1L glass reactor equipped with a mechanical stirrer, a packed column, a reflux condenser, 50g (0.50 mol) of potassium acetate, 200g of 2-pyrrolidone and 300g (1.18 mol) of ethyl 4-acetoxy-2,5,6-trimethylcyclohex-2-ene-1-carboxylate were added. The reaction mixture was stirred and then heated to 155°C while gradually decreasing the internal pressure to 50 mbar. The formed product with acetic acid all together were continuously distilled into a flask over 4h. Then the reaction mass was heated to 165°C while the pressure was decreased to 25 mbar. The distillate was taken up separately into another vessel to be neutralized by aqueous soda followed by washing with water. The subsequent fractionation of the resulting crude oil (223g) at 65-80°C/3 mbar afforded 194g of 2,5,6-trimethylcyclohexa-1,3-diene-1-carboxylate (85% yield). The analysis of the fraction by GC chromatography shows 98% of 2,5,6-trimethylcyclohexa-1,3-diene-1-carboxylate (mix cis + trans 44:56), 0.5% of ethyl 5,6-dimethyl-2-methylenecyclohex-3-ene-1-carboxylate and 1.0% of ethyl 2,5,6-trimethylcyclohexa-2,4-diene-1-carboxylate.
Ethyl (5SR,6RS)-2,5,6-trimethylcyclohexa-1,3-diene-1-carboxylate (Trans isomer):
   H¹-NMR (CDCl₃, 500 MHz) δ 0.92 (d, 3H); 1.01 (d, 3H); 1.31 (t, 3H); 2.11 (m, 1H); 2.14 (s, 3H); 2.62 (m, 1H); 4.22 (q, 2H); 5.78 (d, 1H); 5.94 (m, 1H).
   ¹³C-NMR (CDCl₃, 125MHz) δ 14.4; 18.4; 18.8; 20.8; 35; 35.4; 59.9; 125.3; 127.9; 136.1; 139.9; 168.8.
ethyl (SRS,6RS)-2,5,6-trimethylcyclohexa-1,3-diene-1-carboxylate (Cis isomer):
   H¹-NMR (CDCl₃, 500 MHz) δ 0.84 (d, 3H); 1.09 (d, 3H); 1.31 (t, 3H); 2.13 (s, 3H); 2.63 (m, 1H); 2.64 (m, 1H); 4.22 (q, 2H); 5.65 (d, 1H); 5.84 (m, 1H).
   ¹³C-NMR (CDCl₃, 125MHz) δ 10; 14.4; 15.9; 20.5; 33.1; 33.3; 59.9; 128.5; 130; 137; 141.2; 168.8.

## Claims

1. A process for the preparation of a compound of formula (I) 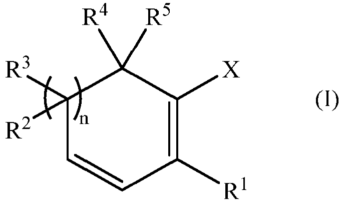 in a form of any one of its stereoisomers or a mixture thereof and wherein n is an integer comprised between 0 and 13; R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group;
comprising the reaction of a compound of the formula (II) 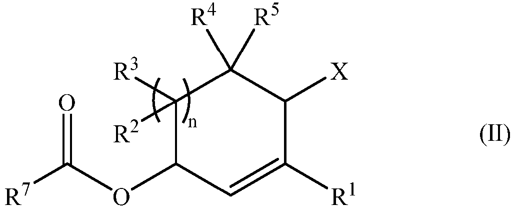 in a form of any one of its stereoisomers and wherein n, X, R¹, R², R³, R⁴ and R⁵ have the same meaning as defined in formula (I) and R⁷ represents a hydrogen atom, a C₂₋₆ alkenyl group, a C₁₋₆ alkyl group optionally substituted by one or more of a halogen atom, or a C₆₋₁₀ aryl group, optionally substituted by one or more of a hydroxy, C₁₋₆ alkyl, C₂₋₆ alkenyl, C₁₋₆ alkoxy group and/or halogen atom;
with a base having a boiling point superior to the boiling point of the compound of formula (I).

2. The process according to claim 1, wherein the base is a metal carboxylate, a metal bicarbonate or a metal carbonate.

3. The process according to claim 2, wherein the metal carbonate is of formula
MₚCO₃ (III)
Wherein M is an alkali metal or an alkaline earth metal and p is 1 or 2; provided that when M is an alkaline earth metal, then p is 1 and when M is a alkali metal, then p is 2.

4. The process according to claim 2, wherein the metal bicarbonate is of formula
M(HOCO₂)_{q} (IV)
wherein M is a alkali metal or a alkaline earth metal and q is 1 or 2; provided that when M is an alkaline earth metal, then q is 2 and when M is a alkali metal, then q is 1.

5. The process according to claim 2, wherein the metal carboxylate is of formula
(RCOO)ᵣM (V)
Wherein R is a hydrogen atom or a C₁₋₁₈ hydrocarbon group; M is an alkali metal or an alkaline earth metal and r is 1 or 2; provided that when M is an alkali metal, then r is 1 and when M is an alkaline earth metal, then r is 2.

6. The process according to claim 5, wherein R is a hydrogen atom, a phenyl group, a C₁₋₁₀ alkyl group or a C₂₋₁₀ alkenyl group, preferably, R is a hydrogen atom, a phenyl group or a C₁₋₆ alkyl group, preferably R is a hydrogen atom, a phenyl group or a C₁₋₃ alkyl group.

7. The process according to according to any one of claims 3 to 6, wherein M is selected from the group of magnesium, calcium, potassium, sodium and lithium, preferably potassium.

8. The process according to according to any one of claims 5 to 7, wherein the process is carried out in the presence of a catalytic amount of the metal carboxylate.

9. The process according to according to any one of claims 1 to 8, wherein n is a integer between 0 to 4; preferably n is 1.

10. The process according to any one of claims 1 to 9, wherein X represents a C(O)R⁶ or COOR⁶ group wherein R⁶ represents a hydrogen atom, a C₁₋₃ alkyl group or a C₂₋₃ alkenyl group.

11. The process according to any one of claims 1 to 10, wherein R² and R³, independently from each other, are a hydrogen atom; R⁴ and R⁵, independently from each other, are a hydrogen atom or a C₁₋₃ alkyl group; R¹ is a hydrogen atom or a C₁₋₃ alkyl group, preferably, R¹ is a methyl or an ethyl group and R⁷ is a methyl, ethyl, propyl, isopropyl or phenyl group.

12. The process according to any one of claims 1 to 11, wherein the compound of formula (I) is removed continuously during the process.

13. The process according to any one of claims 1 to 12, wherein the process is perform in the presence of at least one aprotic dipolar solvent.

14. A compound of formula 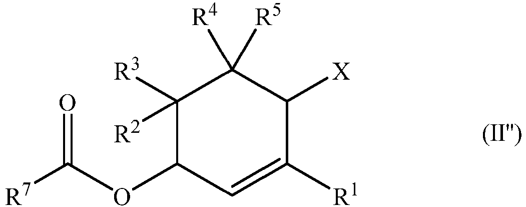 in a form of any one of its stereoisomers and wherein R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group and R⁷ represents a hydrogen atom, a C₂₋₆ alkenyl group, a C₁₋₆ alkyl group optionally substituted by one or more of a halogen atom, or a C₆₋₁₀ aryl group, optionally substituted by one or more of a hydroxy, C₁₋₆ alkyl, C₂₋₆ alkenyl, C₁₋₆ alkoxy group and/or halogen atom; provided that 4-(but-2-enoyl)-3,5,5-trimethylcyclohex-2-en-1-yl acetate and ethyl 4-acetoxy-2,6,6-trimethylcyclohex-2-ene-1-carboxylate are excluded.

15. A compound of formula 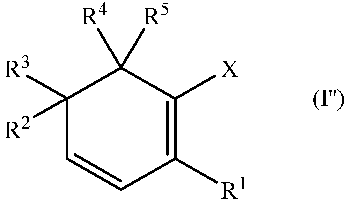 in a form of any one of its stereoisomers or a mixture thereof and wherein R¹, R², R³, R⁴ and R⁵, independently from each other, represent a hydrogen atom, a C₁₋₆ alkyl group or a C₂₋₆ alkenyl group; or R⁴ and R⁵ are taken together and form a C₃₋₆ cycloalkyl group; X is a C(O)R⁶, COOR⁶, CN or C=NR⁶ group wherein R⁶ represents a hydrogen atom, a C₁₋₆ alkyl group or a C₂₋₆ alkenyl group; provided that ethyl 2-ethyl-6,6-dimethylcyclohexa-1,3-diene-1-carboxylate, methyl 2,6,6-trimethylcyclohexa-1,3-diene-1-carboxylate, ethyl 2,6,6-trimethylcyclohexa-1,3-diene-1-carboxylate, 1-(2,6,6-trimethylcyclohexa-1,3-dien-1-yl)ethan-1-one and 1-(2,6,6-trimethylcyclohexa-1,3-dien-1-yl)but-2-en-1-one are excluded.

## Patentansprüche

1. Prozess zur Herstellung einer Verbindung von Formel (I) 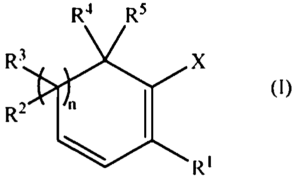
in einer Form eines ihrer Stereoisomere oder einer Mischung davon und wobei n eine ganze Zahl zwischen 0 und 13 ist; R¹, R², R³, R⁴ und R⁵unabhängig voneinander ein Wasserstoffatom, eine C₁₋₆-Alkylgruppe oder eine C₂₋₆-Alkenylgruppe darstellen; oder R⁴ und R⁵ zusammengenommen werden und eine C₃₋₆-Cycloalkylgruppe bilden; X eine C(O)R⁶-, COOR⁶-, CN- oder C=NR⁶-Gruppe ist, wobei R⁶ ein Wasserstoffatom, eine C₁₋₆-Alkylgruppe oder eine C₂₋₆-Alkenylgruppe darstellt;
umfassend die Umsetzung einer Verbindung der Formel (II) 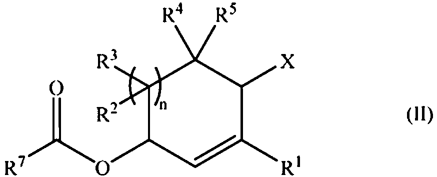
in einer Form eines ihrer Stereoisomere und wobei n, X, R¹, R², R³, R⁴ und R⁵ dieselbe Bedeutung wie in Formel (I) definiert aufweisen und R⁷ ein Wasserstoffatom, eine C₂₋₆-Alkenylgruppe, eine C₁₋₆-Alkylgruppe, gegebenenfalls durch ein oder mehrere Halogenatome substituiert, oder eine C₆₋₁₀-Arylgruppe, gegebenenfalls durch eine oder mehrere Hydroxy-, C₁₋₆-Alkyl-, C₂₋₆-Alkenyl- oder C₁₋₆-Alkoxygruppe substituiert, und/oder ein Halogenatom darstellt;
mit einer Base, die einen Siedepunkt höher als der Siedepunkt der Verbindung von Formel (I) aufweist.

2. Prozess nach Anspruch 1, wobei die Base ein Metallcarboxylat, ein Metallbicarbonat oder ein Metallcarbonat ist.

3. Prozess nach Anspruch 2, wobei das Metallcarbonat die folgende Formel hat
MₚCO₃ (III)
wobei M ein Alkalimetall oder ein Erdalkalimetall ist und p 1 oder 2 ist; vorausgesetzt, dass, wenn M ein Erdalkalimetall ist, p 1 ist und, wenn M ein Alkalimetall ist, p 2 ist.

4. Verfahren nach Anspruch 2, wobei das Metallbicarbonat die folgende Formel hat
M(HOCO₂)_{q} (IV)
wobei M ein Alkalimetall oder ein Erdalkalimetall ist und q 1 oder 2 ist; vorausgesetzt, dass, wenn M ein Erdalkalimetall ist, q 2 ist und, wenn M ein Alkalimetall ist, q 1 ist.

5. Prozess nach Anspruch 2, wobei das Metallcarboxylat die folgende Formel hat
(RCOO)ᵣM (V)
wobei R ein Wasserstoffatom oder eine C₁₋₁₈-Kohlenwasserstoffgruppe ist; M ein Alkalimetall oder ein Erdalkalimetall ist und r 1 oder 2 ist; vorausgesetzt, dass, wenn M ein Alimetall ist, r 1 ist und, wenn M ein Erdalkalimetall ist, r 2 ist.

6. Prozess nach Anspruch 5, wobei R ein Wasserstoffatom, eine Phenylgruppe, eine C₁₋₁₀-Alkylgruppe oder eine C₂₋₁₀-Alkenylgruppe ist, vorzugsweise R ein Wasserstoffatom, eine Phenylgruppe oder eine C₁₋₆-Alkylgruppe ist, vorzugsweise R ein Wasserstoffatom, eine Phenylgruppe oder eine C₁₋₃-Alkylgruppe ist.

7. Prozess nach einem der Ansprüche 3 bis 6, wobei M aus der Gruppe von Magnesium, Calcium, Kalium, Natrium und Lithium, vorzugsweise Kalium ausgewählt ist.

8. Prozess nach einem der Ansprüche 5 bis 7, wobei der Prozess in der Gegenwart einer katalytischen Menge des Metallcarboxylats ausgeführt wird.

9. Prozess nach einem der Ansprüche 1 bis 8, wobei n eine ganze Zahl zwischen 0 und 4 ist; n vorzugsweise 1 ist.

10. Prozess nach einem der Ansprüche 1 bis 9, wobei X eine C(O)R⁶- oder COOR⁶-Gruppe darstellt, wobei R⁶ ein Wasserstoffatom, eine C₁₋₃-Alkylgruppe oder eine C₂₋₃-Alkenylgruppe darstellt.

11. Prozess nach einem der Ansprüche 1 bis 10, wobei R² und R³unabhängig voneinander ein Wasserstoffatom sind; R⁴ und R⁵ unabhängig voneinander ein Wasserstoffatom oder eine C₁₋₃-Alkylgruppe sind; R¹ ein Wasserstoffatom oder eine C₁₋₃-Alkylgruppe ist, vorzugsweise R¹ eine Methyl- oder eine Ethylgruppe ist und R⁷ eine Methyl-, Ethyl-, Propyl-, Isopropyl- oder Phenylgruppe ist.

12. Prozess nach einem der Ansprüche 1 bis 11, wobei die Verbindung von Formel (I) während des Prozesses kontinuierlich entfernt wird.

13. Verfahren nach einem der Ansprüche 1 bis 12, wobei der Prozess in der Gegenwart mindestens eines aprotischen dipolaren Lösungsmittels durchgeführt wird.

14. Verbindung von Formel 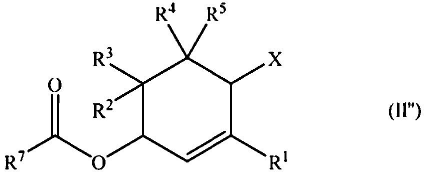 in einer Form eines ihrer Stereoisomere und wobei R¹, R², R³, R⁴ und R⁵unabhängig voneinander ein Wasserstoffatom, eine C₁₋₆-Alkylgruppe oder eine C₂₋₆-Alkenylgruppe darstellen; oder R⁴ und R⁵ zusammengenommen werden und eine C₃₋₆-Cycloalkylgruppe bilden; X eine C(O)R⁶-, COOR⁶-, CN- oder C=NR⁶-Gruppe ist, wobei R⁶ ein Wasserstoffatom, eine C₁₋₆-Alkylgruppe oder eine C₂₋₆-Alkenylgruppe darstellt und R⁷ ein Wasserstoffatom, eine C₂₋₆-Alkenylgruppe, eine C₁₋₆-Alkylgruppe, gegebenenfalls durch ein oder mehrere Halogenatome substituiert, oder eine C₆₋₁₀-Arylgruppe, gegebenenfalls durch ein oder mehrere Hydroxy-, C₁₋₆-Alkyl-, C₂₋₆-Alkenyl- oder C₁₋₆-Alkoxygruppen substituiert, und/oder ein Halogenatom darstellt; vorausgesetzt, dass 4-(But-2-enoyl)-3,5,5-trimethylcyclohex-2-en-1-ylacetat und Ethyl-4-acetoxy-2,6,6-trimethylcyclohex-2-en-1-carboxylat ausgeschlossen sind.

15. Verbindung von Formel 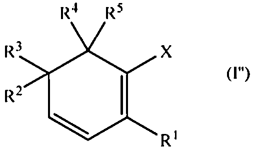 in einer Form eines ihrer Stereoisomere oder einer Mischung davon und wobei R¹, R², R³, R⁴ und R⁵unabhängig voneinander ein Wasserstoffatom, eine C₁₋₆-Alkylgruppe oder eine C₂₋₆-Alkenylgruppe darstellen; oder R⁴ und R⁵ zusammengenommen werden und eine C₃₋₆-Cycloalkylgruppe bilden; X eine C(O)R⁶-, COOR⁶-, CN- oder C=NR⁶-Gruppe ist, wobei R⁶ ein Wasserstoffatom, eine C₁₋₆-Alkylgruppe oder eine C₂₋₆-Alkenylgruppe darstellt; vorausgesetzt, dass Ethyl-2-ethyl-6,6-dimethylcyclohexa-1,3-dien-1-carboxylat, Methyl-2,6,6-trimethylcyclohexa-1,3-dien-1-carboxylat, Ethyl-2,6,6-trimethylcyclohexa-1,3-dien-1-carboxylat, 1-(2,6,6-Trimethylcyclohexa-1,3 -dien-1-yl)ethan-1-on und 1-(2,6,6-Trimethylcyclohexa-1,3-dien-1-yl)but-2-en-1-on ausgeschlossen sind.

## Revendications

1. Processus pour la préparation d'un composé de formule (I) 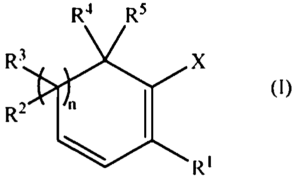
sous forme de l'un quelconque de ses stéréoisomères ou d'un mélange de ceux-ci et dans lequel n est un entier compris entre 0 et 13 ; R¹, R², R³, R⁴ et R⁵, indépendamment les uns des autres, représentent un atome d'hydrogène, un groupe alkyle en C₁₋₆ ou un groupe alcényle en C₂₋₆ ; ou R⁴ et R⁵ sont pris ensemble et forment un groupe cycloalkyle en C₃₋₆ ; X est un groupe C(O)R⁶, COOR⁶, CN ou C=NR⁶, dans lequel R⁶ représente un atome d'hydrogène, un groupe alkyle en C₁₋₆ ou un groupe alcényle en C₂₋₆ ;
comprenant la réaction d'un composé de la formule (II) 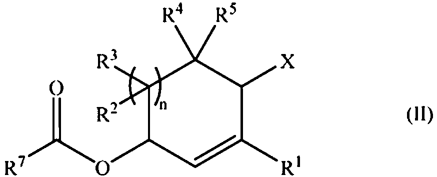
sous forme de l'un quelconque de ses stéréoisomères et dans lequel n, X, R¹, R², R³, R⁴ et R⁵ présentent la même signification que celle définie dans la formule (I) et R⁷ représente un atome d'hydrogène, un groupe alcényle en C₂₋₆, un groupe alkyle en C₁₋₆, éventuellement substitué par un ou plusieurs éléments parmi un atome d'halogène, ou un groupe aryle en C₆₋₁₀, éventuellement substitué par un ou plusieurs éléments parmi un groupe hydroxy, alkyle en C₁₋₆, alcényle en C₂₋₆, alcoxy en C₁₋₆ et/ou un atome d'halogène ;
avec une base présentant un point d'ébullition supérieur au point d'ébullition du composé de formule (I).

2. Processus selon la revendication 1, dans lequel la base est un carboxylate métallique, un bicarbonate métallique ou un carbonate métallique.

3. Processus selon la revendication 2, dans lequel le carbonate métallique est de formule
MₚCO₃ (III)
dans lequel M est un métal alcalin ou un métal alcalino-terreux et p vaut 1 ou 2 ; à condition que lorsque M est un métal alcalino-terreux, alors p vaut 1 et lorsque M est un métal alcalin, alors p vaut 2.

4. Processus selon la revendication 2, dans lequel le bicarbonate métallique est de formule
M(HOCO₂)_{q} (IV)
dans lequel M est un métal alcalin ou un métal alcalino-terreux et q vaut 1 ou 2 ; à condition que lorsque M est un métal alcalino-terreux, alors q vaut 2 et lorsque M est un métal alcalin, alors q vaut 1.

5. Processus selon la revendication 2, dans lequel le carboxylate métallique est de formule
(RCOO)ᵣM (V)
dans lequel R est un atome d'hydrogène ou un groupe hydrocarboné en C₁₋₁₈ ; M est un métal alcalin ou un métal alcalino-terreux et r vaut 1 ou 2 ; à condition que lorsque M est un métal alcalin, alors r vaut 1 et lorsque M est un métal alcalino-terreux, alors r vaut 2.

6. Processus selon la revendication 5, dans lequel R est un atome d'hydrogène, un groupe phényle, un groupe alkyle en C₁₋₁₀ ou un groupe alcényle en C₂₋₁₀, de préférence, R est un atome d'hydrogène, un groupe phényle ou un groupe alkyle en C₁₋₆, de préférence R est un atome d'hydrogène, un groupe phényle ou un groupe alkyle en C₁₋₃.

7. Processus selon l'une quelconque des revendications 3 à 6, dans lequel M est sélectionné dans le groupe composé du magnésium, du calcium, du potassium, du sodium et du lithium, de préférence du potassium.

8. Processus selon l'une quelconque des revendications 5 à 7, dans lequel le processus est réalisé en présence d'une quantité catalytique du carboxylate métallique.

9. Processus selon l'une quelconque des revendications 1 à 8, dans lequel n est un entier compris entre 0 et 4 ; de préférence, n vaut 1.

10. Processus selon l'une quelconque des revendications 1 à 9, dans lequel X représente un groupe C(O)R⁶ ou COOR⁶, dans lequel R⁶ représente un atome d'hydrogène, un groupe alkyle en C₁₋₃ ou un groupe alcényle en C₂₋₃.

11. Processus selon l'une quelconque des revendications 1 à 10, dans lequel R² et R³, indépendamment l'un de l'autre, sont un atome d'hydrogène ; R⁴ et R⁵, indépendamment l'un de l'autre, sont un atome d'hydrogène ou un groupe alkyle en C₁₋₃; R¹ est un atome d'hydrogène ou un groupe alkyle en C₁₋₃, de préférence, R¹ est un groupe méthyle ou un groupe éthyle et R⁷ est un groupe méthyle, éthyle, propyle, isopropyle ou phényle.

12. Processus selon l'une quelconque des revendications 1 à 11, dans lequel le composé de formule (I) est éliminé en continu au cours du processus.

13. Processus selon l'une quelconque des revendications 1 à 12, dans lequel le processus est réalisé en présence d'au moins un solvant dipolaire aprotique.

14. Composé de formule 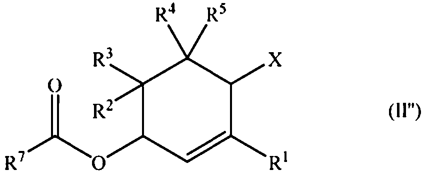 sous forme de l'un quelconque de ses stéréoisomères et dans lequel R¹, R², R³, R⁴ et R⁵, indépendamment les uns des autres, représentent un atome d'hydrogène, un groupe alkyle en C₁₋₆ ou un groupe alcényle en C₂₋₆ ; ou R⁴ et R⁵ sont pris ensemble et forment un groupe cycloalkyle en C₃₋₆ ; X est un groupe C(O)R⁶, COOR⁶, CN ou C=NR⁶, dans lequel R⁶ représente un atome d'hydrogène, un groupe alkyle en C₁₋₆ ou un groupe alcényle en C₂₋₆ et R⁷ représente un atome d'hydrogène, un groupe alcényle en C₂₋₆, un groupe alkyle en C₁₋₆ éventuellement substitué par un ou plusieurs éléments parmi un atome d'halogène, ou un groupe aryle en C₆₋₁₀, éventuellement substitué par un ou plusieurs éléments parmi un groupe hydroxy, alkyle en C₁₋₆, alcényle en C₂₋₆, alcoxy en C₁₋₆ et/ou un atome d'halogène ; à condition que l'acétate de 4-(but-2-énoyl)-3,5,5-triméthylcyclohex-2-én-1-yle et le 4-acétoxy-2,6,6-triméthylcyclohex-2-ène-1-carboxylate d'éthyle soient exclus.

15. Composé de formule 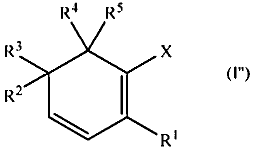 sous forme de l'un quelconque de ses stéréoisomères ou d'un mélange de ceux-ci et dans lequel R¹, R², R³, R⁴ et R⁵, indépendamment les uns des autres, représentent un atome d'hydrogène, un groupe alkyle en C₁₋₆ ou un groupe alcényle en C₂₋₆; ou R⁴ et R⁵ sont pris ensemble et forment un groupe cycloalkyle en C₃₋₆; X est un groupe C(O)R⁶, COOR⁶, CN ou C=NR⁶, dans lequel R⁶ représente un atome d'hydrogène, un groupe alkyle en C₁₋₆ ou un groupe alcényle en C₂₋₆ ; à condition que le 2-éthyl-6,6-diméthylcyclohexa-1,3-diène-1-carboxylate d'éthyle, le 2,6,6-triméthylcyclohexa-1,3-diène-1-carboxylate de méthyle, le 2,6,6-triméthylcyclohexa-1,3-diène-1-carboxylate d'éthyle, la 1-(2,6,6-triméthylcyclohexa-1,3-dién-1-yl)éthan-1-one et la 1-(2,6,6-triméthylcyclohexa-1,3-dién-1-yl)but-2-én-1-one soient exclus.
